(19) Europäisches Patentamt
European Patent Office
Office européen des brevets

(11) Numéro de publication: **0 277 849**
**A1**

# (12) DEMANDE DE BREVET EUROPEEN

(21) Numéro de dépôt: **88400046.4**

(22) Date de dépôt: **11.01.88**

(51) Int. Cl.⁴: **A 61 K 47/00**
A 61 K 9/50

(30) Priorité: **13.01.87 FR 8700264**

(43) Date de publication de la demande:
**10.08.88 Bulletin 88/32**

(84) Etats contractants désignés:
**AT BE CH DE ES FR GB GR IT LI LU NL SE**

(71) Demandeur: **IRE-CELLTARG S.A.**
**Zone Industrielle**
**B-6220 Fleurus (BE)**

(72) Inventeur: **Nicolas, Jean-Marie**
**Moerlaanstraat 3**
**B-1900 Overijse (BE)**

(74) Mandataire: **Warcoin, Jacques et al**
**Cabinet Régimbeau 26, avenue Kléber**
**F-75116 Paris (FR)**

## (54) Procédé d'incorporation d'un ou plusieurs principes actifs lipophiles dans des lipoprotéines, lipoprotéines obtenues et composition pharmaceutique les contenant.

(57) L'invention concerne un procédé d'incorporation d'un ou plusieurs principes actifs lipophiles dans des lipoprotéines.

Selon l'invention, ce procédé comporte les étapes suivantes :(a) les lipoprotéines sont mises en présence d'un détergent ; (b) le mélange obtenu est mis en présence d'un principe actif à incorporer ; (c) le détergent est éliminer.

L'invention concerne également les lipoprotéines dans lesquelles ont été incorporés un ou plusieurs principes actifs par ledit procédé et des compositions pharmaceutiques dans lesquelles ledit principe actif a été incorporé par le procédé.

EP 0 277 849 A1

**Description**

PROCEDE D'INCORPORATION D'UN OU PLUSIEURS PRINCIPES ACTIFS LIPOPHILES DANS DES LIPOPROTEINES, LIPOPROTEINES OBTENUES ET COMPOSITION PHARMACEUTIQUE LES CONTENANT.

La présente invention concerne des compositions pharmaceutiques comportant des lipoprotéines, destinées à véhiculer un ou plusieurs principes actifs liposolubles à l'intérieur de cellules malades ainsi que le procédé permettant d'obtenir de telles compositions.

Ces compositions sont destinées au traitement de nombreuses affections, et, par exemple, au traitement des affections parasitaires, fongiques ou bactériennes.

Le traitement de telles affections est, en effet généralement rendu difficile par la localisation intracellulaire de l'agent infectieux et par l'importante toxicité des agents thérapeutiques disponibles.

La leishmaniose, par exemple, est causée par des protozoaires, les leishmania, qui parasitent les cellules du système réticulo-endothélial. Selon Alvring (1), plus de 12 millions de personnes en sont atteintes. Il existe des composés efficaces contre cette pathologie, mais leur emploi est malheureusement limité par l'importance de leur toxicité.

Ce problème thérapeutique concerne toutes les infections intra-cellulaires du système réticulo-endothélial, qu'elles soient d'origine parasitaire, fongique ou bactérienne.

De façon plus générale, l'un des inconvénients majeurs de la thérapeutique actuelle est la spécificité généralement insuffisante des molécules actives. La plupart d'entre elles agissent sans suffisamment de discernement au niveau de sites trop variés et leur administration se traduit pratiquement toujours par l'apparition d'effets indésirables.

Une façon de résoudre ce problème consiste à faire appel à des véhicles capables de conduire les molécules actives jusqu'aux sites désignés, autrement dit, jusqu'à la seule cible visée. Les qualités indispensables du véhicule sont liées à l'utilisation envisagée : biodégrabilité, innocuité, tropisme, pour la seule cible visée. A cela, s'ajoutent des contraintes industrielles : fabrication à grande échelle, conservation pendant une période suffisant. Divers véhicules ont été envisagés : macromolécules, enveloppes cellulaires, liposomes, nanocapsules...

Or, il s'est avéré que les lipoprotéines ont un tropisme très marqué pour les cellules, et notamment pour les cellules du système réticulo-endothélial. Ce tropisme est lié au circuit du cholestérol dans l'organisme. Les lipoprotéines sont, en effet, des complexes véhiculant le cholestérol dans l'organisme. Ces complexes sont classés selon leur densité en chylomicrons, lipoprotéines à très faible densité (VLDL), lipoprotéines à faible densité (LDL) et lipoprotéines à haute densité (HDL).

Le tropisme cellulaire des lipoprotéines s'explique schématiquement de la façon suivante : pour faire face à leur besoin en cholestérol, les cellules absorbent ces lipoprotéines ; pour ce faire, les lipoprotéines se fixent sur des récepteurs cellulaires ; ceux-ci se meuvent à la surface des cellules jusqu'à des régions déprimées ; ces régions s'invaginent progressivement à l'intérieur du cytoplasme cellulaire et finissent par se refermer sur l'ensemble récepteur-lipoprotéines pour former des vésicules ; ces vésicules migrent dans la cellule ; au cours de cette migration, les récepteurs se séparent des lipoprotéines et retournent à la surface cellulaire ; les lipoprotéines, libérées, fusionnent avec les lysosomes dont l'action enzymatique permet la libération de cholestérol.

Du fait de ce tropisme très marqué pour les cellules les lipoprotéines constituent des vecteurs médicamenteux intracellulaires de choix dans le traitement d'affections pathologiques diverses.

En 1982, Counsell et Coll. (2) ont évoqué la possibilité d'utiliser les LDL comme vecteurs de médicaments. Il est en effet possible de tirer profit de la spécificité cellulaire de ces LDL pour piloter des principes actifs, soit vers le système réticulo-endothélial, soit vers les tissus présentant un métabolisme élevé vis-à-vis du cholestérol.

En outre, les LDL, de par leur structure, devraient permettre d'encapsuler dans des volumes extrêmements faibles (diamètre d'environ 200 Å) une grande variété de composés lipophiles, notamment, les dérivés d'alcaloïde de vinca à chaîne grasse à activité anticancéreuse.

Plusieurs techniques visant à "encapsuler" des composés thérapeutiques dans les lipoprotéines, et notamment les LDL ont été décrites (3). Elles présentent de nombreux inconvénients : rendement d'encapsulation très faible, recours à des solvants organiques avec altération concommittante de l'activité biologique de l'apoprotéine B, recours à des phénomènes de transferts actifs.

Ces inconvénients conduisent, pour la plupart d'entre eux, à l'impossibilité d'utiliser les lipoprotéines comme vecteur intracellulaire. Ainsi, par exemple, l'apoprotéine B est une protéine constitutive de LDL qui constitue le site spécifique de reconnaissance par les récepteur cellulaires à LDL. Une modification de cette protéine, ou une altération de son activité biologique, conduisent bien évidemment à sa non-reconnaissance par les récepteurs celllulaires, et donc à la non-absorption des LDL par les cellules.

La présente invention a permis de développer un procédé d'encapsulation appliquable à n'importe quel type de lipide ou composé liposoluble, et qui n'altère par les propriétés biologiques des lipoprotéines.

Le procédé selon la présente invention consiste essentiellement à incorporer un ou plusieurs principes actifs lipophiles dans les lipoprotéines, en faisant appel à un détergent quelconque.

Selon sa caractéristique essentielle, il comporte les étapes suivantes :

a) les lipoprotéines sont mises en présence du détergent,

b) puis le mélange obtenu est mis en présence du principe actif,

c) enfin le détergent est éliminé.

Le procédé permet notamment d'incorporer un agent thérapeutique liposoluble ou rendu liposoluble par modification chimique dans les lipoprotéines sous perturbation des propriétés biologiques de ces dernières. De plus, l'agent thérapeutique, une fois incorporé, conserve son activité.

On peut citer comme quantités de détergent mis en jeu dans procédé 0,001 mg à 1 mg/mg de lipoprotéine et comme quantités de principe actif 0,001 mg à 5 mg/mg de lipoprotéine.

Toutefois, ces valeurs sont données à titre indicatif et non limitatif dans la mesure où il est toujours possible de trouver des détergents très actifs à faible dose, ceci étant vrai également pour les principes actifs. Inversement, indépendamment d'impératifs économiques, on peut engager des quantités plus importantes en détergent sans modifier le résultat final puisque ce même détergent est éliminé en cours de procédé.

Parmi les avantages notables du procédé selon l'invention, on peut citer :

. sa simplicité qui rend la technique aisément exploitable industriellement,

. le fait qu'il soit applicable à tout principe actif hydrophobe sans qu'il soit nécessaire de recourir à des étapes élaborées de synthèses chimiques,

. le fait qu'il ne soit pas fait usage de solvants organiques, ni d'opérations de centrifugation ou lyophilisation contrairement aux procédés antérieurs, les préparations obtenues étant ainsi mieux adaptées à une utilisation en thérapeutique humaine.

Le mécanisme d'incorporation est complexe. On distingue vraisemblablement deux fonctions différentes du détergent. Ce dernier peut, en effet, avoir pour fonction de dissocier les lipoprotéines ou de perméabiliser leur membrane.

L'un ou l'autre mécanisme interviendra préférentiellement selon d'une part, que la concentration en détergent utilisée est supérieure ou inférieure à la concentration micellaire critique, et d'autre part, que le rapport molaire entre le détergent et les lipoprotéines est plus ou moins important.

En effet, lorsque les molécules de détergent forment des micelles, l'incorporation se fera difficilement à travers la membrane des lipoprotéines et c'est la dissociation qui se produira.

En outre, bien évidemment, si le rapport massique du détergent par rapport aux lipoprotéines est augmenté, la dissociation se produira plus certainement.

Donc, selon un mode particulier de l'invention , notamment pour des concentrations micellaires en détergent, le procédé d'incorporation d'un ou plusieurs principes actifs lipophiles dans des lipoprotéines est caractérisé en ce qu'il comporte les étapes successives suivantes :

(a) les lipoprotéines sont dissociées en leurs éléments par mise en présence d'un détergent,

(b) après dissociation, les éléments obtenus à l'étape (a) sont mis en présence du principe actif à incorporer,

(c) les lipoprotéines sont alors reconstituées, après un éventuel changement ou modification de l'apoprotéine, pour encapsuler le principe actif par élimination du détergent.

Pour des concentrations submicellaires, comme on l'a dit, la dissociation des lipoprotéines n'a pas lieu ou est faible, et l'incorporation se fait à travers la membrane des lipoprotéines.

Bien que les lipoprotéines, plus particulièrement utiles comme vecteurs médicamenteux selon l'invention, soient les LDL, le procédé selon l'invention peut être appliqué pour d'autres lipoprotéines telles que les chylomicrons, les lipoprotéines à très faible densité (VLDL) et les lipoprotéines à haute densité (HDL).

Les éléments des LDL sont essentiellement les esters de cholestérol, des phospholipides et du cholestérol libre ainsi qu'une protéine : l'apoprotéine B.

L'étape a) du présent procédé est mise en oeuvre, de préférence au moyen d'un détergent tel qu'un sel d'un acide stérolique comme le déoxycholate de sodium, ce détergent est dénommé NaDoc dans la suite de la description, l'utilisation d'un détergent ayant une structure voisine du cholestérol étant capable de jouer un rôle stabilisant dans les lipoprotéines.

Cependant, tout détergent ionique ou non ionique, et notamment un détergent moins cher, convient tout aussi bien.

En effet, le constituant détergent, selon l'invention, peut être ionique, non-ionique, ampholytique ou à caractère dipolaire, ou peut être un mélange de ces types. On trouvera de nombreuses listes de tels agents tensio-actifs ioniques, non-ioniques, à caractère dipolaire et amphotère dans la littérature.

Le principe actif incorporé dans les lipoprotéines peut être quelconque mais est, de préférence, choisi parmi les antiparasitaires, antifongiques, antibactériens et/ou anticancéreux. Ce principe actif, lipophile, peut être incorporé directement ou après greffage d'une chaine lipophile sur sa structure.

L'apoprotéine B peut être modifiée chimiquement, par exemple, par acétylation, afin de changer son tropisme ou son activité ou bien peut être changée pour une autre apoprotéine.

De préférence, on utilise des LDL acétylées ; ces dernières ont en effet un tropisme très marqué pour les cellules du système réticulo-endothélial, telles que les macrophages.

Dans l'étape b) la mise en présence des éléments de LDL et du principe actif à encapsuler est effectuées par mise en contact des éléments obtenus, et du détergent avec le principe actif.

Enfin, dans l'étape c), l'élimination du détergent peut être effectuée par toute méthode appropriée, notamment par dialyse.

La présente invention s'étend aux lipoprotéines obtenues après l'incorporation d'une ou de plusieurs principes actifs.

Les lipoprotéines utiles comme vecteurs médicamentaux ont, à l'origine, la composition chimique suivante:

| | VLDL | LDL | HDL |
|---|---|---|---|
| protéines | 7% | 21% | 47% |
| phospholipides | 18% | 23% | 28% |
| triglycérides | 55% | 9% | 7% |
| Cholestérol | 20% | 47% | 18% |

La présente invention concerne plus particulièrement les LDL.

Les LDL (low-density lipoprotein) sont des lipoprotéines présentes dans le plasma humain et responsables du transport du cholestérol. Ce sont des particules sphériques d'un diamètre de 220 Å contenant une partie lipidique centrale composée d'environ 1.500 molécules d'esters à longue chaîne du cholestérol. Cet espace central lipidique est emprisonné à l'intérieur d'une couche sphérique polaire formée d'environ 500 molécules de cholestérol libre, de 800 molécules de phospholipides et d'une protéine : l'apoprotéine B-100.

Les cellules humaines possèdent à leur surface des récepteurs pour les LDL. Une fois lié à son récepteur, le LDL est endocyté et se dégrade dans les lysosomes.

C'est cette apoprotéine B qui est reconnue spécifiquement par les récepteurs cellulaires à LDL, et qui dirige par conséquent les LDL vers des cellules spécifiques. Cette apoprotéine conserve intégralement ses propriétés biologiques après incorporation du ou des principes actifs dans les LDL par le procédé selon l'invention.

Cependant, en vue d'établir un ciblage encore plus spécifique des cellules malades, et notamment, en vue d'atteindre des cellules cancéreuses, on peut modifier chimiquement ladite apoprotéine et ce, préalablement à l'étape c) de reconstitution des lipoprotéines, le cas échéant.

On s'est, en effet, aperçu que, de façon inattendue, au cours de l'étape de dissociation des lipoprotéines, l'apoprotéine se sépare des autres constituants lipidiques des LDL, pour se lier intimement au détergent.

On peut, selon la présente invention, éliminer le détergent ayant incorporé cette protéine, puis recueillir l'apoprotéine par des techniques connues de l'homme de l'art, pour pouvoir modifier chimiquement cette dernière en vue, par exemple, de lui fournir un site supplémentaire de reconnaissance par des récepteurs de cellules particulières, et, notamment, des cellules cancéreuses.

L'apoprotéine, une fois modifiée, peut être réintroduite par l'intermédiaire du détergent avec les autres constituants lipoprotéiniques en vue de la reconstitution des LDL ayant éventuellement incorporé le ou les principes actifs.

C'est pourquoi, la présente invention concerne aussi des lipoprotéines dont on a volontairement modifié l'apoprotéine B.

Dans le cas du NaDoc, la concentration mi cellaire critique est de l'ordre de 2 mM.

On observera un mécanisme de dissociation pour un rapport en poids de détergent par rapport aux lipoprotéines de 10 à 30, le mécanisme de perméabilité intervenant plutôt pour des rapports en poids de l'ordre de 1 à 3.

La présente invention concerne, enfin, les compositions pharmaceutiques destinées à véhiculer un ou plusieurs principes actifs liposolubles et comportant des lipoprotéines dans lesquelles ledit principe actif a été incorporé par le procédé selon la présente invention. Le principe actif véhiculé peut être quelconque ; il est en particulier un composé antifongique ou anticancéreux. Par exemple, il peut s'agir des dérivés du kétoconazole ou d'alcaloïde de vinca décrits dans les demandes de brevet français nos 86 01496, 86 00364 et 86 17412.

La mise en oeuvre préférée du procédé selon la présente invention ressortira de la description qui va suivre et en se référant aux dessins annexés. Sur ces dessins :

- La figure 1 représente le schéma qui semble le plus probable concernant le processus de dissociation-reconstitution des LDL. Sur cette figure:

▩ représente le détergent
■ représente l'apoprotéine B
▨ représente les phospholipides et cholestérol libre
▤ représente les esters de cholestérol et triglycérides

- La figure 2 représente la chromatographie sur sephadex G 200, équilibrée avec le tampon D, de la préparation LDL-NaDoc (débit = 0,1 ml ; volume d'élution = 2 ml).

L'apoprotéine B (●), le cholestérol (■), et les phospholipides (▲) sont ensuite dosés sur chaque

fraction.

- La figure 3 représente l'électrophorèse sur agarose 1 % des LDL (A) et des LDL traitées au NaDoc (B).

- La figure 4 représente l'Ouchterlowny de l'apoprotéine B (6,8,10,12) vis à vis d'un sérum anti-apoprotéine B.

- La figure 5 représente le SDS-PAGE Pharmacia PAA 4/30 ;
gradient = 0,2 % SDS
1, 2, 3 = LDL
4, 5, 6 = apoprotéine B.

- La figure 6 représente les :

A) chromatographie sur sephadex G 200, équilibré avec le tampon c, des LDL reconstituées. (Débit - 0,1 ml/min; volume de fraction = 2 ml).
Le cholestérol (●), les phospholipides (■) et l'apoprotéine B (▲) sont ensuite dosés sur chaque fraction.

B) Droite d'étalonnage du dosage de l'apoprotéine B.

- La figure 7 représente l'électrophorèse sur agarose 1 % LDL natives (A) et des LDL reconstituées (B).

- La figure 8 représente l'immunoélectrophorèse sur agarose 1,5 % des LDL natives (A), des LDL reconstituées (B) et des HDL (C) vis-à-vis d'un sérum anti-apoprotéine B.

- La figure 9 représente la chromatographie sur sephadex G 200, équilibré avec le tampon C, des LDL reconstituées cholestérol- $[^3H]$ . (Débit = 0,1 ml/min ; volume de fraction = 2 ml). Le cholestérol (○), l'apoprotéine B (●) et la radio-activité (Δ) sont mesurés sur chaque fraction.

- La figure 10 représente la chromatographie sur sephadex G200, équilibré avec le tampon C, des LDL reconstituées oléate de cholestérol- $[^{14}C]$ (LDL rec. O. CHoL. $[^{14}C]$). Débit = 0,1 ml/min ; volume de fraction = 2 ml. Le choles térol (●), la radioactivité (▲) et l'apoprotéine B sont ensuite dosés sur chaque fraction (●).

- La figure 11 représente l'accumulation des LDL rec O.CHOL $[^{14}C]$ (○) et des AC-LDL rec O.CHOL $[^{14}C]$ (●) par les macrophages J774 G8. Les cellules sont rincées à l'aide de milieu RPMI puis incubées à 37° C avec ce même milieu contenant 10 µg/ml de LSL rec O.CHOL $[^{14}C]$ ou d'AC-LDL rec.O.CHOL $[^{14}C]$. Après incubation aux temps indiqués, la radioactivité acide trichloracétique soluble est déterminée dans les surnageants. La radioactivité associée aux cellules est également mesurée.

- La figure 12 représente l'incorporation d'un agent thérapeutique par le processus de dissociation-reconstitution des LDL.
Sur cette figure :

▦ représente l'apoprotéine B
▧ représente les phospholipides et le cholestérol libre
▩ représente les esters de cholestérol et les triglycérides
■ représente le détergent
☒ représente l'agent thérapeutique

- La figure 13 représente le % de récupération obtenu au terme du processus de reconstitution pour

A) l'incorporation de l'agent CTO400A
B) l'incorporation de l'agent CTO40013.

- La figure 14 représente la chromatographie des LDL rec CTO40013 sur sephadex G 200 équilibrée avec le tampon B. Débit = 0,1 ml/min ; volume de fraction = 2 ml. Le cholestérol (●), l'apoprotéine B (Δ) et les phos pholipides (○) sont ensuite dosés sur chaque fraction, ainsi que le composé CTO40013 (●).

- La figure 15 représente l'influence du plasma sur le relarguage de la molécule incorporée. Les LDL rec O.CHOL $[^{14}C]$ (A) et les LDL rec CTO40013 (B) sont incubées en présence de plasma pendant 3 heures à 37° C. Les échantillons sont fractionnés sur gradient discontinu en KBr. Les fractions sont récupérés par ordre décroissant de densité.

Le procédé selon la présente invention a notamment permis d'incorporer certains composés thérapeutiques dans des LDL acétylées dont les récepteurs cellulaires correspondants sont portés par les macrophages J 774 G8 comme il sera décrit ci-après.

- La figure 16 représente la solubilisation du

$C_{91}$Base

en présence de différents milieux d'incubation. 100 µL d'une solution éthanolique en $C_{91}[^3H]$ (activité spécifique : 1026 dpm.µgr$^{-1}$ ; 1 mgr.mL$^{-1}$) sont évaporés sous Argon. Le résidu sec est mis en présence de 2 mL de tampon C + LDL (500 µgr.mL$^{-1}$) + NaDOC (2mM) (●); de 2 mL de tampon C + LDL (500 µgr.mL$^{-1}$) (○) ; de 2 mL de tampon C + NaDOC (2mM) (■) et de 2 mL de tampon C (□). Les préparations sont incubées à 25° C sous agitation modérée. La radioactivité présente dans le surnageant est déterminée après différents temps d'incubation.

- La figure 17 représente la disparition PLASMATIQUE et accumulation HEPATIQUE des $^{125}$I LDL

natives (○) et des $^{125}$I LDL-$C_{91}$ (●).

- La figure 18 représente la biodistribution des $^{125}$I LDL$_{natives}$ (□) et $^{125}$I LDL-$C_{91}$ (■). La CAPTURE TISSULAIRE RELATIVE est déterminée une heure après injection. 25 µgr de LDL sont injectés dans la veine de la queue de souris Balb/c de +/- 20 gr.

- La figure 19 représente la cytotoxicité des LDL-$C_{91}$ (A) et du

$C_{91}$ sel (B)

sur Fibroblastes Humains.

## I - ETUDE DES INTERACTIONS ACETYL-LDL-MACROPHAGES

### 1) Caractéristiques des LDL

Les LDL apparaissent en microscopie électronique comme des particules sphériques et homogènes d'un diamètre approximatif de 200 Å. Leur altération (causée par exemple par vieillissement) déclenche un phénomène d'agrégation, les LDL s'agrégeant en "piles d'assiettes".

Les études d'activité biologique des LDL, ici menées, sont effectuées sur des macrophages en lignée continue, les J774 G8, ceux-ci possédant exclusivement des récepteurs pour les LDL chimiquement modifiées par acétylation. Cette modification chimique est reflétée par une variation morphologique. En microscopie électronique, les LDL acétylées ne se présentent plus comme des particules sphériques mais prennent une forme ovalaire. Ce phénomène est totalement différent d'un processus d'altération des LDL.

### 2) Caractéristiques des macrophages

Les cellules utilisées comme modèle pour l'étude des récepteurs à acétyl-LDL sont des cellules de souris provenant de lymphome histiocytaire maintenues en lignée continue ; à savoir les macrophages J774 G8. Cette lignée présente de nombreuses caractéristiques communes avec les macrophages péritoneaux. Les J774 G8 peuvent reconnaître spécifiquement les acétyl-LDL et les métaboliser. L'ensemble des résultats présentés montrent que ce processus de métabolisation est en tout point semblable à celui décrit par Brown lors de son étude sur les macrophages péritoneaux (4).

### 3) Acétylation des LDL

L'acétylation selon la technique de Basu et Coll (5) conduisant à une précipitation irréversible de plus de 70 % des LDL, une méthode alternative plus douce permettant de ramener à 17% cette précipitation est mise au point.

#### a) Principe

L'anhydride acétique est un agent capable d'induire l'acétylation des LDL, celle-ci se produisant au niveau des résidus ε-$NH_2$ des fonctions lysines de l'apoprotéine B.

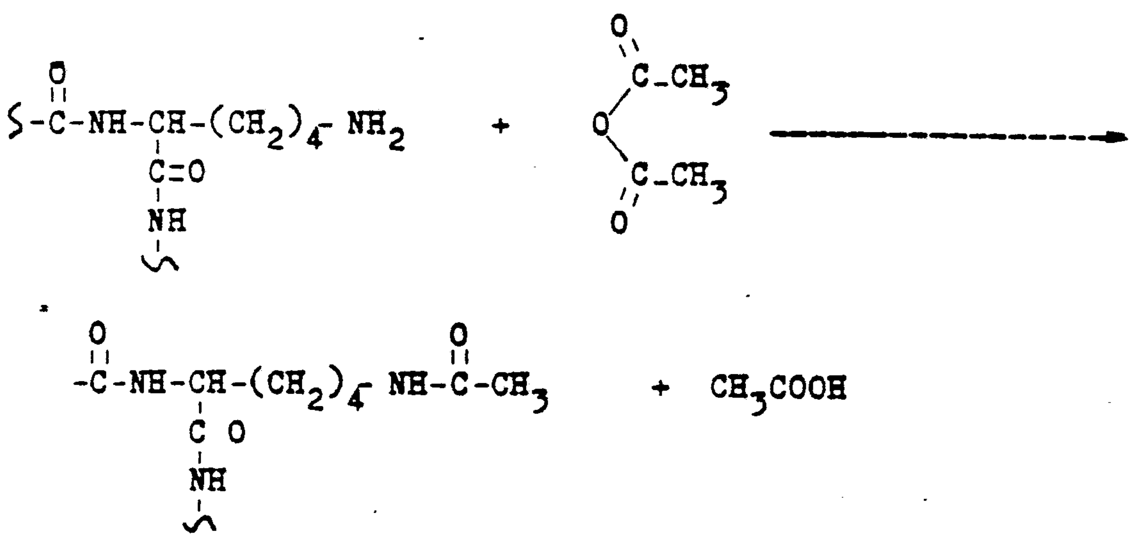

b) Acétylation à froid

A 1 ml d'une préparation de LDL (5 mg), on ajoute 1 ml d'une solution d'acétate de sodium saturée. L'ensemble est maintenu à 4° C sous agitation constante. L'anhydride est alors additionné par aliquots de 2 μl, ceci toutes les 15 minutes. Le pH contrôlé en permanence et maintenu à pH 8 par adjonction de NaOH 0,1 M. Le pourcentage de lysine modifiée étant suivi par la réaction à la fluorescamine,(la fluorescamine réagit directement avec les fonctions amines primaires pour former un complexe fluorescent), le processus d'acétylation est stoppé lorsque le pourcentage indique 45 % de résidus lysines modifiées.

c) Acétylation au tritium

On ajoute 1 ml d'une solution d'acétate de sodium saturée à 1 ml de la préparation de LDL (5 mg). L'anhydride acétique tritié (100 mCi) est ensuite ajouté, et la préparation ainsi marquée, maintenue à 4° C sous agitation. On poursuit l'acétylation par adjonction d'anhydride acétique froid comme il est décrit.

Suite à l'acétylation, les LDL perdent leur forme sphérique pour prendre un aspect "ovalaire". Ce phénomène est totalement différent d'un processus de dégradation des LDL, qui, lui, se traduit par une agrégation en "piles d'assiettes".

L'obtention d'acétyl-LDL radio-marquées s'effectue par acétylation des LDL à l'aide d'anhydride acétique tritié. Au terme de la réaction, la préparation est dialysée contre un tampon B (NaCl 0,15 M, $KH_2PO_4$ 10 mM, pH 7,4) de façon à ramener le pourcentage de radioactivité acide trichloracétique soluble à 1 %.

4) Interaction à 37° C des acétyl-LDL avec les macrophages J774 G8

Lorsque les macrophages J774 G8 sont incubés en présence d'acétyl-LDL tritiées (10 μg/ml) à 37° C, la radioactivité associée aux cellules augmente au cours du temps pour atteindre un plateau après 2 heures. Les produits de digestion sont déjà présents dans le milieu de culture après 30 minutes et augmentent de façon linéaire jusqu'à un temps de 5 heures.

En présence de chloroquine, le processus de digestion est bloqué, ce qui entraine une augmentation de la concentration intracellulaire en acétyl-LDL non digérées. La capture (obtenue par la somme de la digestion et de l'accumulation) reste inchangée quelque soit la concentration en chloroquine appliquée. Ceci démontre le rôle des lysosomes dans le processus de digestion.

5) Etude des récepteurs à acétyl-LDL des macrophages J774 G8

Afin de caractériser les récepteurs à acétyl-LDL, des acétyl-LDL tritiées ont fait l'objet d'une étude de l'accumulation. L'analyse des résultats de Scatchard révèle l'existence de deux types de sites de fixation : des sites à haute affinité et des sites aspécifiques à faible affinité.

c) Etudes de déplacement des acétyl-LDL tritiées

Il est intéressant d'observer dans quelle mesure les LDL natives peuvent interagir avec les récepteurs à acétyl-LDL présents sur les macrophages J774 G8. A cette fin, on procède à des expériences de compétition où l'on étudie l'influence des acétyl-LDL et des LDL sur le métabolisme des acétyl-LDL tritiées par les J774 G8.

On constate qu'un excès molaire en acétyl-LDL de l'ordre de 20 suffit pour diminuer de 86 % l'accumulation des acétyl-LDL tritiées. Par contre, les LDL n'exercent pas cet effet de compétition.

En effet, présentes en un excès molaire de 20, elles nentrainent qu'une diminution de 10 % de l'accumulation des acétyl-LDL tritiées. Les mêmes observations peuvent être faites en ce qui concerne l'effet des acétyl-LDL sur la digestion des acétyl-LDL tritiées.

7) Conclusion

L'ensemble des résultats présentés ci-dessus montre que les acétyl-LDL se fixent sur des récepteurs spécifiques présents en surface des macrophages J774 G8. Ces récepteurs reconnaissent exclusivement les LDL acétylées comme le montrent les expériences de compétition.

Les acétyl-LDL fixées sont ensuite transportées vers les lysosomes où elles sont digérées. En effet, si on inhibe les enzymes lysosomiaux à l'aide de chloroquine, on ne retrouve plus de produits de digestion dans le milieu extracellulaire (6).

Au niveau des lysosomes, l'apoprotéine B est décomposée en acides aminés. Les acides gras et le cholestérol sont libérés et, par la suite, incorporés dans la membrane cellulaire.

II - PROCESSUS DE DISSOCIATION - RECONSTITUTION DES LDL (figure 1)

1) Dissociation des LDL par le NaDoc

Les LDL isolées sur sepharose CL-4B sont dialysées pendant 24 heures à 4° C contre un tampon C ($Na_2CO_3$ 50 mM ; NaCl 50 mM ; pH 10). Le NaDoc est ajouté à la préparation à raison de 2,5 mg par mg de LDL.

Après 30 minutes d'incubation à température ambiante, l'échantillon est chromatographié sur séphadex G 200 préalablement équilibré avec le tampon D ($Na_2CO_3$ 50 mM, NaCl 50 mM ; NaDoc 10 mM pH 10) incluant 10 mM de NaDoc (Figure 2).

La composition protéinique élue dans le volume mort de la colonne alors que les lipides éluent dans le volume total, la récupération en apoprotéine B étant de 92 %.

Les LDL sont testées en électrophorèse sur agarose 1 % avant et après adjonction de NaDoc (figure 3). Le

traitement des LDL par le NaDoc conduit à la formation de micelles mixtes. Cette dissociation est démontrée en électrophorèse par l'apparition d'une bande diffuse ne correspondant plus à une migration caractéristique des LDL.

Il est à remarquer que si la dissociation est effectuée à pH neutre, ou si la quantité de NaDoc ajoutée est ramenée à 1 mg par mg de LDL, l'apoprotéine B reste associée aux esters de cholestérol et aux triglycérides.

L'apoprotéine B récupérée par le procédé décrit ci-dessus conserve son activité antigénique vis à vis d'un antisérum anti-apoprotéine B humain (figure 4).

Afin de mettre en évidence d'éventuelles altérations de l'apoprotéine B, celle-ci est testée en SDS-PAGE. Après coloration, on observe une seule bande correspondant à un poids moléculaire de 316.200. Ceci est en accord avec les résultats de Chapman et coll. (Figure 5) (7).

Notons que l'apoprotéine B recueillie est complexée au NaDoc. Il est possible d'éliminer simplement ce NaDoc par chromatographie un séphadex G200 (ceci en absence de détergent).

Les fractions contenant l'apoprotéine B ne présentent pas de précipités visibles, toutefois, une centrifugation à 40.000 tpm pendant une heure conduit à la précipiation de l'apoprotéine B.

2) Obtention de LDL reconstituée par enlèvement de détergent

La méthode repose sur la dissociation des LDL en micelles mixtes comme décrit en 1). Il suffit dans une seconde étape d'éliminer le détergent de ces micelles de sorte que les différents lipides et l'apoprotéine B se réassocient en particules homogènes. Les LDL sont dissociées en présence de NaDoc (voir 1). Ce dernier est éliminé par dialyse contre cinq fois un litre de tampon C. L'utilisation concommittante de NaDoc-[$^{14}$C] montre que dans ces conditions, plus de 99 % du détergent est éliminé. La préparation est ensuite redialysée contre un tampon B.

3) Caractérisation des LDL reconstituées

Les LDL reconstituées sont chromatographiées sur sephadex G200 en présence d'un tampon C. On constate que la composante protéinique est éluée en même temps que les différents composants forment un complexe macromoléculaire stable (figure 6).

En électrophorèse sur agarose 1 %, ce complexe présente une migration de type β semblable à celle des LDL natives (figure 7). De même, en immunoélectrophorèse, le comportement des LDL reconstituées est en tout point semblable à celui des LDL natives (figure 8). L'analyse en microscopie électronique démontre clairement l'homogénéité de la préparation des LDL reconstituées, leur diamètre moyen étant de 200 Å.

4) Interactions LDL reconstituées ~ JEG

Afin de vérifier que le processus de dissociation-réassociation ne perturbe pas la reconnaissance des lipoprotéines par les récepteurs cellulaires, les LDL reconstituées sont incubées avec des cellules de choriocarcinome de type JEG. Ces cellules présentent en surface des récepteurs à LDL natives. Le test est volontairement limité à une étude à 37°C en fonction du temps d'incubation, la digestion apparaissant déjà après 30 minutes et restant linéaire jusqu'à 20 heures.

5) Incorporation de lipides radiomarqués dans les LDL par la technique de reconstitution

a) Incorporation de cholestérol - [$^{3}$H]

1 ml de LDL (1 mg/ml) sont dialysées pendant 24 heures à 4°C contre un litre de tampon C. On ajoute à cette préparation 400 µl d'une solution de NaDoc à 20 mg/ml et 4 µl d'une solution de cholestérol tritié (4,64 mg par ml de méthanol). Après 2 heures d'agitation à t° ambiante, l'échantillon est dialysé contre le tampon C.

Afin de mesurer dans quelle proportion le cholestérol tritié participe au processus de réassociation, l'échantillon est chromatographié sur séphadex G200 en présence d'un tampon C (figure 9). On observe que 90 % de la radioactivité appliquée lors de la reconstitution se retrouve dans le volume mort, associée à l'apoprotéine B et au cholestérol. Les LDL reconstituées ainsi obtenues présentent toutes les caractéristiques physico-chimiques et biologiques des LDL natives.

b)Incorporation d'oléate de cholestérol- [$^{14}$C]

Un schéma identique à celui décrit en II- 5 a) est appliqué à l'incorporation d'oléate de cholestérol-[$^{14}$C] (figure 10). Après incorporation, l'échantillon est divisé en deux fractions. La première subit une acétylation à froid, puis est dialysée 24 heures à 4°C contre le tampon B (Ac-LDL rec. O.CHOL.[$^{14}$C]).

La seconde fraction ne subit pas de modification (LDL rec. O.CHOL. [$^{14}$C]).

En électrophorèse sur agarose 1 %, les LDL rec. O.CHOL. [$^{14}$C], migrent de façon identique aux LDL natives.

Les Ac-LDL rec.O.CHOL. [$^{14}$C] n'étant pas assez concentrées pour être détectées par la coloration, le gel d'agarose est découpé en languettes de 2 mm, chaque languette faisant par la suite l'objet d'un comptage [$^{14}$C]. On peut ainsi confirmer l'augmentation de la migration dans le cas de l'acétylation.

Il est connu que les macrophages et notamment les J774 G8 accumulent les esters de cholestérol présents dans les LDL, se transformant ainsi en cellules "mousseuses". Ce phénomène est observé pour peu que les LDL soient modifiées chimiquement, par exemple, par acétylation. Les macrophages J774 G8 sont incubés à 37°C en présence de LDL rec.O.CHOL.[$^{14}$C] (figure 11).

Dans le cas de la préparation acétylée, la radioactivité associée aux cellules augmente de façon linéaire au cours du temps, ce qui traduit bien une accumulation cellulaire des esters de cholestérol. La préparation non acétylée n'étant pas reconnue par les récepteurs, ne présente pas cette caractéristique. Dans les deux cas, il n'est pas possible de détecter la radioactivité acide trichloracétique soluble dans les milieux extracellulaires. On peut donc en conclure que la digestion ne concerne pas les esters de cholestérol et que ces derniers s'accumulent dans le cytoplasme.

6) Conclusion

Comme il a été décrit dans l'introduction, les LDL renferment approximativement 80 % de lipides dont des esters de cholestérol du cholestérol libre, des phospholipides et des triglycérides. On a démontré qu'un détergent comme par exemple le NaDoc pouvait désorganiser la structure des LDL. Lors de ce processus, le NaDoc, détergent ionique relativement faible, s'associe à l'apoprotéine B et aux autres composés lipidiques des LDL pour former des micelles mixtes, l'apoprotéine B n'étant pas dégradée lors de cette opération.

En outre, la structure quaternaire n'est en rien modifiée puisque, après ce traitement, l'apoprotéine B, maintient son activité biologique. On peut poser l'hypothèse que le NaDoc, ayant une structure proche du choles térol, se substitue au cholestérol présent dans l'environnement immédiat de l'apoprotéine B. Cette technique permet donc de purifier l'apoprotéine B sous forme soluble et sans altération notable.

On a pu également démontrer le caractère réversible de cette dissociation. En effet, par simple élimination du détergent, on récupère des LDL intactes, présentant toutes les caractéristiques des LDL de départ. Ceci confirme le fait que les LDL répondent à une structure thermodynamiquement favorisée.

Il est donc possible de tirer profit du processus de reconstitution pour incorporer un lipide exogène.

III - APPLICATION DU PROCESSUS DE DISSOCIATION-RECONSTITUTION DES LDL POUR L'INCORPORATION D'UN AGENT ANTIFONGIQUE (figure 12)

Les composés utilisés sont les suivants :
Le CTO400A qui est un agent antifongique, et
Le CTO400I 13 qui est un dérivé acide gras (oléate) du composé CTO4001.

1) Incorporation d'un agent antifongique dans les LDL

Le procédé d'incorporation décrit ci-dessus est appliqué à un agent antifongique :
le CT0400A ( kétoconazole : cis-4-(4-(2,4-dichloro-phényl)-2-(1H-imidazol-1-yl-méthyl)-1,3 dioxolan-4-yl méthoxy)phényl)-1-(acétyl) pipérazine ).
Afin d'évaluer l'influence du caractère lipophile du médicament sur son degré "d'incorporabilité", le CT04000A est comparé à son dérivé acide gras
le CT040013 (oléate de kétoconazole : cis-4-(4-(2,4-dichlorophényl)-2-(1H-imidazol-1-yl-méthyl)-1,3 dioxolan-4-yl méthoxy)phényl)-1-(oléoyl)pipérazine).

On ajoute à 1,6 mg de LDL préalablement dialysées contre le tampon C, 10 mg de NaDoc solide et on laisse réagir 30 minutes à température ambiante. On ajoute à la préparation 2,8 ml d'une solution NaDoc 20 mM, $Na_2CO_3$ 50 mM, NaCl 50 mM, pH 10 contenant 482 µg de CT4000A ou 482 µg de CTO400I3. Les échantillons sont dialysés à 4°C contre trois fois 1 litre de tampon B.

Le pourcentage de récupération en LDL, en CTO400I3 et en NaDoc est déterminé au terme du processus de reconstitution (figure 13).

Il est également possible de rapporter la masse de substance incorporée par mole de LDL. Les résultats obtenus pour le CTO4000A et la CTO400I3 sont respectivement de 0,4 et de 208 moles incorporés par mole de LDL.

2) Caractérisation des LDL reconstituées en pré sence de l'agent CTO400I3 (LDL rec.CTO400I3)

Il était intéressant de montrer dans le cas des LDL rec. CTO400I3 que le composé thérapeutique était réellement associé à la lipoprotéine. La préparation a donc été chromatographiée sur sephadex G 200 (figure 14), et on peut voir que le composé CTO400I3 est élué en même temps que les différents composants des LDL. On constate en outre que la composition chimique des LDL rec. CTO400I3 n'a pas été modifiée lors du processus d'incorporation (tableau I).

TABLEAU 1

Influence de la reconstitution sur la composition chimique des LDL.
La composition des LDL est donnée en % du poids total.

| | Apoprotéine B | Phospholipides | Triglycérides | cholestérol |
|---|---|---|---|---|
| LDL natives | 21 | 23 | 9 | 47 |
| LDL Rec. CTO40013 | 24 | 25 | 8 | 43 |

Une analyse de ces LDL rec. CTO40013 en électrophorèse sur agarose 1 % et en OUchterlowny montre que le profil électrophorétique et que la réactivité antigénique sont préservées.

3) Etude de la stabilité des LDL rec. CTO400I3 en présence de plasma

Dans un milieu biologique aussi complexe que le plasma, de nombreux constituants peuvent, à priori, inte) ragir avec les LDL rec. CTO400I3 et provoquer leur déstabilisation ou entraîner une libération du principe actif incorporé. Aussi, une première étude a été entreprise pour essayerde caractériser le comportement des LDL rec. CTO400I3 lorsque ces dernières sont mises en présence de plasma.

Deux préparations ont été testées : les LDL rec. O.CHOL [$^{14}C$] et les LDL rec. CTO400I3. 500 µl de ces différentes préparations sont incubées avec 1 ml de plasma humain pendant trois heures à 37° C. Au terme de cette incubation, les échantillons sont fractionnés par ultracentrifugation sur gradient discontinu en KBr. Par aspiration, on récupère les fractions correspondantes aux différentes densités, et sur chacune de celle-ci, le cholestérol, les phospholipides, l'oléate de cholestérol [$^{14}C$] sont dosés (figure 15). Le profil en cholestérol total et en phospholipides est identique dans les deux gradients. On y distingue trois pics correspondants aux trois classes de lipoprotéines, par ordre décroissant de densité : HDL, LDL, et VLDL.

Dans le cas des LDL rec.O.CHOL. [$^{14}C$], il y a une redistribution complète de la redioactivité. En effet, on constate que l'oléate de cholestérol est transféré des LDL vers les HDL et VLDL. Ceci est en accord avec les études Pattnaik (8) où il est démontré que les lipoprotéines échangent leurs lipides, ces échanges étant catalysés par des protéines de transfert.

Le composé CTO400I3, par contre, n'est pas pris en charge par ces protéines de transfert. L'albumine sérique présente la caractéristique d'adsorber les composés liposolubles. Lors de cette étude, il n'a pas été possible de mettre en évidence une association du CTO400I3 et de l'albumine. On peut donc supposer que la drogue est bien localisée dans le noyau apolaire des LDL et ne peut être mise en contact avec l'albumine.

4) Discussion

Ces résultats montrent que parmi les paramètres capables d'influencer l'incorporation d'une substance thérapeutique dans les LDL, le caractère lipophile de cette substance joue un rôle majeur. En effet, par simple greffage d'une chaîne d'acide gras à la molécule CTO4000A, il est possible d'augmenter son pourcentage d'incorporation d'un facteur 500.

L'utilisation des LDL in vivo comme vecteurs potentiels de principes actifs nécessite qu'avant d'atteindre les cellules cibles, l'intégrité structurale soit conservée au contact des liquides biologiques.

Ces essais montrent qu'en présence de plasma, le principe actif incorporé dans les LDL n'est pas relargué. On peut donc avancer l'hypothèse d'un modèle où le principe actif est localisé dans le moyau des LDL et reste ainsi à l'abri de l'influence des autres protéines plasmatiques.

Cependant, les explications et hypothèses énoncées ci-dessus ne limitent en rien la portée de la présente invention. Celle-ci a donc permis de réaliser de nouveaux vecteurs médicamenteux permettant de véhiculer un principe actif lipophile jusqu'à la cellule cible visée. Ce principe actif peut être incorporé directement dans les lipoprotéines ou aprés greffage d'une chaine lipophile.

IV) - UTILISATION DE CONCENTRATIONS SUBMICELLAIRES EN DETERGENT POUR L'INCORPORATION D'AGENTS THERAPEUTIQUES LIPOSOLUBLES DANS LES LDL

1. Méthode

Les LDL isolées sur sépharose CL-4B sont dialysées pendant 24 heures à 4° C contre un tampon C.

L'agent thérapeutique est solubilisé dans un solvant organique à raison de 1 mg par ml. 100 µL de cette solution sont évaporés sous Argon dans un tube à essai. On ajoute au résidu sec, 2 ml de la solution de LDL et du NaDOC de façon à atteindre une concentration finale de 1 à 5 mM selon l'agent thérapeutique considéré. La préparation est incubée à 25°C pendant 6 heures sous agitation modérée.

Au terme de l'incubation, l'échantillon est filtré sur un filtre Millipore 0,45 µm. Le NaDOC est ensuite éliminé par chromatographie sur gel P6 (Biorard®) préalablement équilibré avec un tampon C.

La concentration en ApoB et en agent thérapeutique est déterminée sur chaque fraction.Les fractions correspondant aux LDL sont mises en commun et dialysées contre un tampon TrisHCl 10 mM NaCl 0,15 M EDTA 0,3 mM pH 7,4 pendant 24 heures à 4°C.

2. Application à l'incorporation d'un agent cytotoxique

L'ensemble du procédé décrit en IV-1 a été appliqué à un dérivé VINCA ALCALOIDE liposoluble : le "$C_{91}$ base": (4-désacétyl-vinblastine C-3N-dodécyl-carboxamide). La concentration en NaDOC utilisée lors de l'incubation a été ajustée à 2 mM final.

L'examen de la figure 16 montre clairement que seule la combinaison détergent + LDL permet la solubilisation de l'agent thérapeutique. En effet, en absence de NaDOC, les LDL tendent à a'aggréger de façon irréversible. Il est vraisemblable que le détergent perméabilise la membrane lipoprotéinique et facilite ainsi l'entrée de l'agent thérapeutique.

Au cours de tout le processus, les LDL conservent leur intégrité comme le démontre des analyses électrophorétiques en agarose 1 %.

Cette méthode diffère donc fondamentalement de la méthode d'incorporation décrite plus haut même si dans les deux cas, on fait appel à un détergent.

Si on précipite sélectivement les LDL, plus de 99 % du $C_{91}$ base se retrouve dans le culot. On confirme ainsi

que l'agent thérapeutique est bien associé aux LDL. On appellera cette préparation : LDL-$C_{91}$.

Le nombre de molécules de drogue incorporées par molécule de LDL varie selon le temps d'incubation, selon la concentration en NaDOC utilisée lors du procédé et selon le type de drogue envisagée. Dans le cas du $C_{91}$ base, pour une concentration de 2 mM en NaDOC et un temps d'incubation de 60 heures (3 heures à 25° C + 57 heures à 4°C), ce rapport est de 70.

## V - EFFET DE L'INCORPORATION SUR LA DISTRIBUTION TISSULAIRE DES LDL

Les techniques d'incorporation décrites dans la littérature conduisent généralement à une altération des propriétés biologiques des LDL (Masquelier M. et coll. (10)). Cela se traduit in vivo, par une réduction du temps de demi-vie plasmatique et par une accumulation accrue dans le foie et la rate.

### 1. Méthode

Afin d'évaluer la technique d'incorporation décrite IV-1), la biodistribution des $^{125}I$ LDL-$C_{91}$ a été comparée à celles des $I^{125}$ LDL natives.

Les LDL et LDL-$C_{91}$ sont marquées selon la technique de Langer et coll. L'étude de distribution tissulaire est faite sur des souris Balb/c de 20 g. Chaque animal reçoit par injection i.v. dans la queue 25 µg $^{125}I$ LDL (0,5-1 µCi) dans 0,2 ml d'une solution à 0,90 % NaCl. Après 1 heure, le sang est collecté par la veine fémorale et l'animal est décapité. Les organes sont prélevés, rincés dans du PBS et pesés. La capture tissulaire relative est calculée en divisant la radioactivité par gramme d'organe par la radioactivité totale dans le plasma au moment du sacrifice (figure 18). On estime que le volume plasmatique correspond à 4 % du poids total de l'animal.

### 2. Résultats

La figure 17 illustre la disparition plasmatique et l'accumulation hépatique des deux préparations. A ce niveau, il n'y a pas de différence significative entre les deux types de LDL testées. La concentration plasmatique chute rapidement durant la première heure îet décroft ensuite selon une cinétique d'ordre 1 avec un temps de demi-vie de 5 à 6 heures. Plus de 95 % de la radioactivité plasmatique est précipitable en présence d'acide trichloracétique. Parallèlement, les LDL s'accumulent dans le foie pour atteindre au maximum 10 % de la dose injectée. La figure 18 montre clairement que le pattern de distribution est identique pour les deux préparations. On constate que l'incorporation n'a pas de répercussion sur l'accumulation hépatique et splénique des LDL.

## VI - TEST IN VITRO DE CYTOXICITE DES LDL-$C_{91}$

### 1. Méthode

Des fibroblastes humains provenant d'une biopsie de peau sont mis en culture dans des plaques 96 puits à raison de 1000 cellules/puits (Jour 0). Milieu de culture : DMEM 10 % sérum de veau foetal, glutamine, antibiotiques. Les cellules sont maintenues dans un incubateur (5 % $CO_2$/95 % air) à 37°C. Au jour 1, les milieux sont éliminés et remplacés par du milieu frais + agent cytotoxique à différente dilution.

Au jour 2, les milieux sont éliminés, les cellules sont lavées et réincubées dans du milieu frais. Au jour 5, le nombre de cellules vivantes est déterminé par le test au MTT (tétrazolium) selon la technique de Mosmann. Les résultats sont exprimés en logit du pourcentage en cellules survivantes par rapport aux cellules contrôles en fonction du logarithme de la concentration en agent cytotoxique (figure 19).

### 2. Résultats

La $LD_{50}$ (dose létale pour 50 % des cellules) pour les LDL-$C_{91}$ sel est respectivement de 378 et 979 $ng.ml^{-1}$. On constate donc que le $C_{91}$ sous forme incorporée dans les LDL conserve son activité cytotoxique. Dans ces conditions, des LDL natives sont sans effet sur la croissance cellulaire.

La $LD_{50}$ pour les LDL-$C_{91}$ sel est respectivement de 378 et 979 $ng.ml^{-1}$. On constate donc que le $C_{91}$ sous forme incorporée dans les LDL conserve son activité cytotoxique. Dans ces conditions, des LDL natives sont sans effet sur la croissance cellulaire.

## VII - LES LIPOPROTEINES A FAIBLE DENSITE (LDL) COMME VECTEUR DE MEDICAMENTS DANS LE TRAITEMENT DE LA LEISHMANIOSE HUMAINE

Les parasites du genre Leishmania sont transmis par une mouche (forme promasigote) et se développent chez leur hôte exclusivement dans les macrophages tissulaires sous forme d'amastigotes. Les parasites se multiplient au site d'infection (Leishmaniose cutanée : L. tropica & L. mexicana, mais peuvent également migrer via les macrophages circulant au niveau des muqueuses faciales (Leishmaniose mucocutanée : L. braziliensis) ou de certains organes (Leishmaniose viscérale : L. donovani).

Les macrophages de l'hôte assurent le développement et la propagation des parasites. Il a été montré par ailleurs que les LDL modifiés sont prélevés préférentiellement par les macrophages tissulaires. Les vésicules lipidiques représentent ainsi un vecteur potentiel de médicaments dans le traitement de la leishmaniose.

Nous avons montré en utilisant le modèle expérimental de la leishmaniose humaine que :

1. l'activité de l'agent thérapeutique est augmentée sensiblement par son incorporation dans les LDL modifiés ;

2. l'accumulation des LDL modifiés par les macrophages est stimulée par l'infection.

1 - Modèle expérimental in vitro

Le modèle in vitro utilisé, consiste à développer les formes intracellulaires (amastigotes) de Leishmania mexicana anazonentis dans des macrophages péritonéaux de souris Balb/c injectés initialement par les formes promastigotes.

La culture de macrophages est obtenue à partir des cellules de la cavité péritonéale.

Brièvement, la cavité péritonéale est lavée plusieurs fois avec du milieu RPMI 1640 contenant de la pénicilline (100 U/ml) et de la streptomycine (100 µg/ml) additionné de glutamine (2 mM) et de 20 % de sérum de veau foetal décomplémenté à la chaleur. Ce milieu constitue le milieu de culture complet. Après centrifugation de la suspension cellulaire ainsi obtenue, les cellules sont resuspendues dans le même milieu, puis incubées 1 heure à 37° C en présence de 5 % $CO_2$ dans des boîtes de Pétri. Les macrophages adhérents sont ensuite lavés plusieurs fois, détachés de leur support puis comptés. Un volume cellulaire contenant 2,5 x $10^5$ macrophages est ajouté dans chaque puits (16 mm de diamètre) des boîtes de culture contenant chacun une lamelle en verre. Les cellules sont ainsi cultivées 3 jours à 37° C. Avant d'être utilisées, les cultures sont lavées 2 fois pour retirer les cellules non adhérentes. Dans ces conditions, les cultures contiennent 1,5 à 2,0 x $10^5$ macrophages.

La souche de L. mexicana amazonentis est maintenue sous forme promastigote à 27° C par passage successif dans du milieu Schneider Drosophila contenant 100 µg/ml de gentamycine, 2 mM de glutamine, 5 µg/ml d'hémine et additionné de 20 % de sérum de veau foetal décomplémenté.

Une suspension de promastigotes en phase stationnaire est ajoutée à chaque culture de macrophages à raison de 4 parasites par cellule. Les cultures sont incubées à 34,5°-35° C (5 % $CO_2$-95 % air) pendant 7 heures. Les cultures infectées sont lavées avec du milieu sans sérum jusqu'à ce qu'il y ait moins de un parasite par 20 macrophages. Une culture (1 boîte à 4 puits) est fixée et colorée au May-Grûnwald Giemsa pour contrôler l'infection (jour 0). S'il y a moins de 50 organismes intracellulaires par 100 macrophages, l'infection est considérée trop faible pour une interprétation correcte des résultats. 24 heures après l'infection, le milieu de culture est remplacé par du milieu frais contenant ou non un agent thérapeutique. Nous avons choisi d'utiliser le dérivé oléoyl-kétoconazole seul ou incorporé à des LSL natives ou acétylées. Le milieu (± l'agent thérapeutique) est renouvelé au jour 3. Au jour 6, des cultures de macrophages infectés sont fixées, colorées, séchées, la lamelle de verre est ensuite montée sur une lame pour l'observation microscopique.

Le nombre d'amastigotes contenu dans 100 à 200 macrophages est déterminé pour chaque culture. Le nombre moyen d'organismes par 100 macrophages au jour 6 dans les cultures traitées est exprimé en pourcentage du nombre moyen d'organismes par 100 macrophages dans les cultures contrôles au jour 6 (% de survie). Les cultures contrôles contiennent en moyenne 300 à 400 parasites par 100 macrophages ; 60 % en moyenne des macrophages sont infectés et contiennent de 5 à 7 amastigotes.

2 - Résultats

L'activité antileishmanique, à savoir le pourcentage d'inhibition des formes intracellulaires, du dérivé oléoyl-kétoconazole (CI40013 ou KO1) et de ce dérivé incorporé à des LDL natives (LDL-KO1) ou acétylée (AcLDL-KO1) est présentée dans le tableau 2.

Le dérivé oléoyl kétoconazole présente une bonne activité antileishmanique. 50 % d'inhibition ($ED_{50}$) sont obtenus à la dose de 1,4 µg KO1.ml et 90 % d'activité est observée à la dose de 11 µg/ml. Par comparaison au kétoconazole, l'index thérapeutique du KO1 (défini comme le rapport de $ED_{50}$ kéto sur $ED_{50}$ KO1) est de 9 (dose efficace pour 50 % de réduction en parasite).

Lorsque le composé est incorporé à des LDL natives, il perd toute son activité (similaire au contrôle).

Par contre, si les LDL sont modifiées, par exemple par acétylation, l'activité thérapeutique est maintenues et accrue par rapport au KO1 (un facteur 1,6). Près de 90 % de la croissance des parasites est inhibée à la dose de 2 µg KO1/ml.

Il apparaît donc que l'incorporation d'un composé actif dans une vésicule lipidique prélevée préférentiellement par les macrophages influence positivement son activité sur les Leishmanias intracellulaires dans le modèle expérimental in vitro.

Tableau 2 : Activité antileishmanique du dérivé Oléoyl-kétoconazole libre in incorporé dans des LDL natives ou acétylées

| Composé (a) | dose (µg/ml) | Nombre d'amastigotes (b) (% des contrôles) | Activité (c) (% inhibition) |
|---|---|---|---|
| Contrôle | - | 100 (11) | 0 |
| KO1 | 0,5 | 72 (6) | 28 |
| | 1,0 | 57 (3) | 43 |
| | 1,4 | 50 (4) | 50 |
| | 2,0 | 44 (2) | 56 |
| | 2,5 | 36 (6) | 64 |
| | 11,0 | 10 (4) | 90 |
| LDL-KO1 | 2,0 | 95 (5) | 5 |
| AcLDL-KO1 | 2,0 | 10 (2) | 90 |

(a): Les composés sont incubés à 35° en présence de macrophages infectés par L. mexicana amazonentis pendant 6 jours selon la procédure décrite précédemment. Le dérivé KO1 est incorporé aux LDL dans le rapport molaire de 225:1 et aux AcLDL dans le rapport molaire de 164:1.

(b): Les résultats représentent le nombre de parasite par 100 macrophages et sont exprimés en pourcentage du nombre de parasites dans les contrôles du jour 6 (% de survie) (moyennes ± déviation standard de 4 valeurs).

(c) L'activité antileishmanique est représentée par le pourcentage d'inhibition des parasites.

VIII - UTILISATION DES LIPOPROTEINES DE DENSITE LEGERE (LDL) COMME VECTEUR D'ANTIFONGIQUE

Plusieurs espèces de fungi pathogènes pour l'homme peuvent infecter leur hôte soit dans les espaces extracellulaires, soit en survivant et même en se multipliant au sein des cellules, principalement les phagocytes professionnels. Ces parasites intracellulaires facultatifs induisent un état de portage chez l'homme donnant lieu à des épisodes infectieux à répétition. Les antifongiques encapsulés dans les LDL ou Ac-LDL (acétylée) peuvent efficacement être utilisés dans le traitement de sujets souffrant de tels microorganismes (Candida, Cryptococcus, Coccioïdes ...). Nous démontrons ci-après leur avantage thérapeutique tant du point de vue toxicité qu'activité, en utilisant comme dérivés imidazoles le kéto oléïque (KO1) et le kétoconazole-alanine-alanine oléïque (KAAO1) inclus dans des LDL ou Ac-LDL.

1. Tests d'activité dans un modèle intracellulaire d'infection

1.1. Modèle d'infection intracellulaire

La lignée continue de macrophages J77468 d'origine murine provient d'un clonage de la souche J774 ATCC, et est cultivée en milieu RPMI 1640 additionné de glutamine 2 mM, 10 % de sérum de veau foetal (décomplémenté 60 min. à 56°) et de 10 mM Hepes (pH 7.2). Une suspension de 5 $10^6$ cellules/ml sont infectées avec une culture de Candida tropicalis (5 $10^5$ levures/ml) provenant d'une culture de nuit dans de la casitone. Le milieu d'infection utilisé est du RPMI contenant 2 % de sérum de veau nouveau-né et 10 mM Hepes (pH 7.2).

Aprés 3 h d'incubation à 37° dans un mélange d'air 10 % $CO_2$, la phagocytose des levures est complète. La suspension cellulaire macrophages-Candida est centrifugée, lavée abondamment et répartie à raison de $10^5$ macrophages infectés/alvéole dans des boîtes à 24 alvéoles contenant une lamelle de verre (diamètre 14 mm).

Après 3 h de phagocytose (To), sur 100 macrophages, 32 contiennent de 1 à 6 Candida/macrophage, l'infection moyenne étant de 2 Candida/macrophage.

Toutes les levures ont été phagocytées sauf rares exceptions (estimation au microscope optique après coloration au May-Grünwald Giemsa). Douze concentrations différentes des drogues de référence et des drogues encapsulées dans les LDL sont distribuées dans les boîtes contenant les macrophages infectés.

L'évaluation de l'efficacité d'une drogue se fait à chaque concentration. Testées après 18 h d'incubation (37°, dans un mélange air-10 % $CO_2$), les levures se multiplient d'abord au sein des cellules hôtes, puis dans le milieu extracellulaire. Le taux de macrophages infectés est alors de 75 % chez les témoins. Une cellule peut contenir de 1 à plus de 20 Candida/cellule, la majorité contient de 2 à 6 levures/cellule. Le milieu de culture contient $10^8$ Candida extracellulaires/ml (comptage dans une cellule de Burker).

1.2. Détermination de l'activité des drogues encapsulées dans les LDL et Ac-LDL sur des macrophages infectés

La sensibilité des Candida vis-à-vis des antifongiques dépend fortement des conditions expérimentales, aussi l'évaluation d'une drogue doit être menée dans diverses conditions.

L'activité d'une drogue encapsulée ou non dans les LDL ou Ac-LDL par rapport à un antifongique de référence, le kétoconazole (K), est déterminé par la concentration micromolaire en drogue réduisant de 50 % le nombre de macrophages infectés par les Candida (=C150). Signalons que parallèlement à la réduction au cours du traitement, du taux de cellules infectées, le nombre de Candida par macrophage infecté diminue fortement (de 1 à 3 levures/cellule).

1.3.Résultats

L'activité de KO1 encapsulé dans des LDL ou Ac-LDL mesurée dans un milieu contenant 2 % de sérum de veau foetal ou du plasma humain figure aux tableaux 3 et 4. L'activité d'un autre antifongique KAA01 encapsulé dans les LDL est montrée au tableau 4.

Les concentrations en drogue indiquées sont celles du dérivé kétoconazole encapsulé. Le rapport molaire étant en général de environ 1 entre le dérivé K et l'apoprotéine B du LDL ou Ac-LDL.

Tableau 3 : Activité du KO1 encapsulé dans les LDL ou les AcLDL sur des macrophages infectés par *C. tropicalis*

| Antifongique (= A.F.) | CI50 | CI50 AF/AF encapsulé | CI50 K/AF |
|---|---|---|---|
| K | 52,26 ± 38 | - | 1 |
| KO1 | 85,30 ± 38 | - | 0,6 |
| LDL + KO1 | 131,00 ± 96 | 0,6 | 0,4 |
| LDL - KO1 | 1,25 ± 0,47 | 68,2 | 41,8 |
| AcLDL - KO1 | 0,66 | 129,2 | 79,2 |

Le milieu de culture est RPMI + glutamine 2 mM + Hepes 10 mM + 2 % de sérum de veau foetal.

Tableau 4 : Activité du KO1 et du KAAO1 encapsulé ou non dans des LDL sur des macrophages infectés par *C. tropicalis* en présence de plasma humain

| Antifongique (A.F.) | Concentration plasma humain % | CI50 | CI50 AF/AF encapsulé | CI50 K/AF |
|---|---|---|---|---|
| K | 2 | 15,14 | - | 1 |
| KO1 | 2 | 252 | - | 0,06 |
| KAAO1 | 2 | 7,94 | - | 1,91 |
| LDL-KO1 | 2 | 0,06 | 4.200 | 252 |
| LDL-KAAO1 | 2 | 0,10 | 79,4 | 151 |
| LDL-KO1 | 10 | 0,05 | 5.040 | 308 |

2. Toxicité des antifongiques (AF) encapsulés ou non dans des LDL

La viabilité des macrophages J77468 cultivés dans du milieu contenant l'agent antifongique encapsulé ou non dans les LDL figure au Tableau 5. K et KO1 sont toxiques aux concentrations nécessaires pour réduire de 50 % l'infection intracellulaire (C1250) contrairement au LDL-KO1 pour lequel aucune toxicité n'a pu être détectée.

Tableau 5 : Toxicité du K, KO1 et LDL-KO1 pour les macrophages J77468

| Concentration µM | % de viabilité cellulaire* | | |
|---|---|---|---|
| | K | KO1 | LDL-KO1 |
| 100,00 | 1,75 | 38,94 | - |
| 50,00 | 3,50 | 71,22 | - |
| 25,00 | 3,16 | 67,71 | - |
| 12,50 | 16,40 | 69,47 | - |
| 6,25 | 50,52 | 60,70 | - |
| 3,13 | 64,56 | 67,36 | - |
| 1,56 | 86,67 | 67,72 | - |
| 1,22 | -** | - | 95,52 |
| 0,78 | 85,16 | 67,72 | - |
| 0,66 | - | - | 98,40 |
| 0,33 | - | - | 95,85 |
| 0,16 | - | - | 111,50 |
| 0,08 | - | - | 109,90 |
| 0,02 | - | - | 112,00 |

Les conditions de culture sont décrites au Tableau 3. La viabilité cellulaire des macrophages est mesurée après 18 h de culture en présence des AF par la réduction de sels de trétrazolium M.T.T. en un précipité formozan.
* % de réduction du MTT par rapport au témoin (100 %) non traité
** non déterminé

3. Activité des dérivés AF encapsulés sur l'infection extracellulaire

L'action des AF encapsulés sur une infection extracellulaire peut être évaluée de deux manières : d'une part en déterminant le nombre de Candida extracellulaires dans un modèle d'infection intracellulaire (voir VIII § 1.1.), d'autre part en mesurant la concentration minimale inhibitrice (CMI) de ces composés par la méthode de dilutions successives dans des boîtes à multipuits (milieu RPMI).

3.1. Infection extracellulaire dans un modèle de macrophages infectés

Les résultats figurent au Tableau 6.

Tableau 6 : Action des antifongiques encapsulés ou non dans des LDL sur l'infection extracellulaire de macrophages infectés par C. tropicalis

| Antifongique (AF) | addition de | CIext50[a] µM | CIext50 AF / LDL-AF | CIext50 K / AF |
|---|---|---|---|---|
| K | 2 % P1.H.[b] | 0,1 | - | |
| KO1 | | 50,12 | - | 0,002 |
| KAAO1 | | 3,2 | - | 0,030 |
| LDL-KO1 | | 0,03 | 1.670 | 3,330 |
| LDL-KAAO1 | | 2,63 | 1,22 | 0,038 |
| K | 2 % SVF[c] | 0,1 | - | |
| KO1 | | 8,31 | - | 0,012 |
| LDL-KO1 | | 1 | 8,3 | 0,1 |

Les conditions expérimentales sont semblables à celles décrites au Tableau 3 et dans le VIII § 1.1.
a : Clext50 = concentration inhibant de 50 % l'accroissement de population de Candida dans le milieu extérieur
b : P1.H. = plasma humain
c : SVF = sérum de veau foetal

3.2. CMI du LDL.KO1 pour Candida tropicalis et Candida albicans

LDL-KO1 et AcLDL-KO1 peuvent présenter une activité supérieure au KO1 sur C. tropicalis et surtout sur C. albicans (facteur de 1 à 6 fois) dans un milieu de culture pourvu de sérum de veau foetal. Cette activité est plus importante sur C. albicans en présence de plasma humain.

BIBLIOGRAPHIE

1 Alvring, C.R., Steck, E.A., Hanson, W.L., Loizeaux, P.S., Chapman, N.L., Waits, U.B. : "Thérapie améliorée contre la leishmaniose expérimentale par utilisation d'un médicament à base d'antimoine encapsulé dans un liposome", Life Sci., 22 : 1021-1026 (1978)

2 Counsell R.E., Pohland R.C. : "Lipoprotéines, système potentiel de délivrance au niveau d'un site spécifique, comme agents diagnostiques et thérapeutiques", J. Med. Chem., 25 : 1115-1120 (1982)

3 Krieger M., Brown M.S., Faust J.R., Goldstein J.L. : "Remplacement des esters de cholestérol endogène des LDL par du linoléate de cholestérol exogène", J. of Biol. Chem., 253 : 4093-4101 (1978)

4 Goldstein J.L., Ho Y.K., Basu S.K., Brown M.S. : "Site de liaison par fixation aux macrophages et dégradation des LDL acétylées, production massive de dépôt de cholestérol", Proc. Natl. Acad. Sci. U.S.A., 76 : 333-337 (1979)

5 Basu S.K., Goldstein J.L., Anderson R.G.W., Brown M.S. : "Dégradation des LDL cationisées et régulation du métabolisme du cholestérol dans les fibroblastes homozygotes de familles hypercholestérolémiques", Prec. Natl. Acad. Sci. U.S.A., 73 : 3178-3182 (1976)

6 Brown M.S., Suzanna E. Dana, J.L. Goldstein : "Hydrolyse récepteur-dépendante des esters de cholestérol contenus dans les LDL plasmatiques", Proc. Nat. Sci. U.S.A., 72 : 2925-2929 (1975)

7 Chapman M.J., Kane J.P. : "Stabilité de l'apoprotéine de la LDL sérique humaine. Absence d'activité de l'endopeptidase endogène", B.B.R.C., 66 : 1030-1036 (1975)

8 Pattnaik N.M., Montes A., Hughes L.B., Zilversmit D.B., "Protéine d'échange de l'ester de cholestérol dans le plasma humain, BBA, 530 : 428-438 (1978)

9 WALTERS K.A., FLORENCE A.T., DUGARD P.H. Int. J.of Pharmaceutics 10 (1982), 153-163
10 MASQUELIER M., VITOLS S., PETERSON C. Cancer Research 46 (1986), 3842-3847.

**Revendications**

1. Procédé d'incorporation d'un ou plusieurs principes actifs lipophiles dans des lipoprotéines, caractérisé en ce qu'il comporte les étapes suivantes :
(a) les lipoprotéines sont mises en présence d'un détergent ;
(b) le mélange obtenu est mis en présence d'un principe actif à incorporer ;
(c) le détergent est éliminer.
2. Procédé d'incorporation d'un ou plusieurs principes actifs lipophiles dans des lipoprotéines, selon la revendication 1, caractérisé en ce qu'il comporte les étapes successives suivantes :
(a) les lipoprotéines sont dissociées en leurs éléments par mise en présence d'un détergent ;
(b) après dissociation, les éléments obtenus à l'étape (a) sont mis en présence du principe actif à incorporer ;
(c) les liproprotéines sont alors reconstituées après un éventuel changement ou modification de l'apoprotéine pour incorporer le principe actif par élimination du détergent.
3. Procédé selon l'une des revendications 1 ou 2, caractérisé en ce que les lipoprotéines sont des LDL.
4. Procédé selon l'une des revendications précédentes, caractérisé en ce que la quantité de détergent mis en jeu est comprise entre 0,001 mg et 1 mg/mg de lipoprotéine, et la quantité de principe actif mis en jeu est comprise entre 0.001 mg et 5 mg/mg de lipoprotéine.
5. Procédé selon l'une des revendications précédentes, caractérisé en ce que les lipoprotéines sont des LDL dans lesquelles l'apoprotéine B est modifiée chimiquement.
6. Procédé selon l'une des revendications 1 à 5, caractérisé en ce que les lipoprotéines sont des LDL acétylées.
7. Procédé selon la revendication 6, caractérisé en ce que l'acétylation des LDL est effectuée à l'aide d'anhydride acétique.
8. Procédé selon l'une des revendications précédentes, caractérisé en ce que le détergent est choisi parmi les détergents ioniques et non ioniques.
9. Procédé selon l'une des revendications 1 à 8, caractérisé en ce que le détergent est un sel d'un acide stérolique.
10. Procédé selon l'une des revendications 1 à 9, caractérisé en ce que le détergent est du NaDOC.
11. Procédé selon l'une des revendications 1 à 10, caractérisé en ce que le principe actif lipophile est choisi parmi les antifongiques, les antiparasitaires et/ou les antibactériens.
12. Procédé selon l'une des revendications 1 à 10, caractérisé en ce que le principe actif est un anticancéreux.
13. Procédé selon la revendication 11, caractérisé en ce que le principe actif est un dérivé du kétoconazole.
14. Procédé selon la revendication 12, caractérisé en ce que le principe actif est un dérivé d'alaloïde de Vinca.
15. Procédé selon l'une des revendications 1 à 14, caractérisé en ce qu'on greffe une chaîne lipophile sur le principe actif préalablement à son incorporation dans les lipoprotéines.
16. Lipoprotéines dans lesquelles ont été incorporés un ou plusieurs principes actifs par le procédé selon l'une des revendications précédentes.
17. Lipoprotéines dans lesquelles ont été incorporés un ou plusieurs principes actifs selon la revendication 16, caractérisées en ce qu'elles comportent en outre une apoprotéine B ayant subi des modifications.
18. Composition pharmaceutique destinée à véhiculer au moins un principe actif liposoluble, caractérisée en ce qu'elle comporte des lipoprotéines dans lesquelles ledit principe actif a été incorporé par le procédé selon l'une des revendications précédentes.
19. Composition selon la revendication 18, caractérisée en ce que le principe actif est un antifongique, un antiparasitaire et/ou un antibactérien.
20. Composition selon la revendication, 18 caractérisée en ce que le principe actif est un anticancéreux.

Office européen des brevets

# RAPPORT DE RECHERCHE EUROPEENNE

Numero de la demande

EP 88 40 0046

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,X<br>Y | JOURNAL OF MEDICINAL CHEMISTRY, vol. 25, no. 10, octobre 1982, pages 1115-1120, American Chemical Society, US; R.E. COUNSELL et al.: "Lipoproteins as potential site-specific delivery systems for diagnostic and therapeutic agents"<br>* Page 1117, colonne 2, alinéa 2; page 1119, résumé *<br>--- | 1-20 | A 61 K 47/00<br>A 61 K 9/50 |
| Y | CHEMICAL ABSTRACTS, vol. 89, no. 21, 20 novembre 1978, page 433, résumé no. 177840n, Columbus, Ohio, US; E.R. PODACK et al.: "Binding of desoxycholate, phosphatidylcholine vesicles, lipoprotein and of the S-protein to complexes of terminal complement components", & J. IMMUNOL. 1978, 121(3), 1025-30<br>* En entier *<br>--- | 1,9,10 | |
| X | JOURNAL OF MEDICINAL CHEMISTRY, vol. 27, no. 8, août 1984, pages 1037-1043, American Chemical Society, US; R.A. FIRESTONE et al.: "Selective delivery of cytotoxic compounds to cells by the LDL pathway"<br>* Pages 1038-1039 *<br>--- | 1-3,12, 20 | DOMAINES TECHNIQUES RECHERCHES (Int. Cl.4)<br>A 61 K |
| A | EP-A-0 162 129 (K.E. THEURER)<br>* Page 4, alinéa 3; revendications *<br>--- -/- | 1-20 | |

Le présent rapport a été établi pour toutes les revendications

| Lieu de la recherche | Date d'achèvement de la recherche | Examinateur |
|---|---|---|
| LA HAYE | 12-04-1988 | BERTE M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 88 40 0046

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | JOURNAL OF PHARMACEUTICAL SCIENCES, vol. 74, no. 12, décembre 1985, pages 1259-1264, American Pharmaceutical Association, Washington, DC, US; J. SEKI et al.: "Plasma lipoproteins as drug carriers: pharmacological activity and disposition of the complex of beta-sitosteryl-beta-D-glucopyranoside with plasma lipoproteins" * Page 1264 * | 1-3 | |
| X | WO-A-8 607 540 (ONCHOLAB) * Page 3, lignes 21-30; page 4, lignes 5-23; page 7, alinéa 4; revendications * | 1-3,11-14,16,5,18-20 | |

DOMAINES TECHNIQUES RECHERCHES (Int. Cl.4)

Le présent rapport a été établi pour toutes les revendications

| Lieu de la recherche | Date d'achèvement de la recherche | Examinateur |
|---|---|---|
| LA HAYE | 12-04-1988 | BERTE M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)